**Europäisches Patentamt**

**European Patent Office**

**Office européen des brevets**

(19)

(11) Veröffentlichungsnummer: **0 378 824 B1**

(12) **EUROPÄISCHE PATENTSCHRIFT**

(45) Veröffentlichungstag der Patentschrift: **24.02.93**

(51) Int. Cl.5: **A61K 31/20**, A61K 35/60

(21) Anmeldenummer: **89123355.3**

(22) Anmeldetag: **18.12.89**

(54) **Verwendung von Omega-3-Fettsäuren als Antikachexiemittel.**

(30) Priorität: **14.01.89 DE 3901048**

(43) Veröffentlichungstag der Anmeldung:
**25.07.90 Patentblatt 90/30**

(45) Bekanntmachung des Hinweises auf die
Patenterteilung:
**24.02.93 Patentblatt 93/08**

(84) Benannte Vertragsstaaten:
**AT CH DE FR GB IT LI**

(56) Entgegenhaltungen:
EP-A- 0 115 419       EP-A- 0 232 501
EP-A- 0 298 293       EP-A- 0 311 091
EP-A- 0 335 550       WO-A-88/02221
GB-A- 2 084 172

(73) Patentinhaber: **CHIMICASA GMBH**
**Wiesentalstrasse 81**
**CH-7000 Chur(CH)**

(72) Erfinder: **Deininger, Rolf, Dr.**
**Fürst-Pückler-Strasse 44**
**W-5000 Köln 41(DE)**
Erfinder: **Tisdale, Michael John, Dr.**
**Wellcott Star Lane**
**Chaverdon Warwickshire CV35 8 LW(GB)**

(74) Vertreter: **Hach, Hans Karl, Dr.**
**Tarunstrasse 23**
**W-6950 Mosbach-Waldstadt (DE)**

**Beschreibung**

Kachexie, nämlich Auszehrung, führt dazu, daß davonbetroffene Personen abmagern, obwohl ausreichende Mengen an qualifizierter Nahrung zugeführt werden. kachexie ist ein trotz normaler Nahrungsaufnahme extremer Gewichtsverlust durch Reduktion des gesamten Körperfettes und der trockenen Muskelmasse. Kachexie tritt vor allem im Zusammenhang mit bestimmten Tumoren und Aidsinfektionen auf. Es sind derzeit keine wirksamen Maßnahmen bekannt, Kachexie experimentell oder gar klinisch zu beeinflussen.

Aufgabe der Erfindung ist es deshalb, ein zur Behandlung von Kachexie geeignetes Mittel zur Verfügung zu stellen.

Die Erfindung löst diese Aufgabe durch Verwendung von Omega-3-Fettsäuren enthaltendem Fischöl zur Herstellung eines Antikachektikums zur Behandlung von Tumorkachexie (Kachexia cancerativa).

Aus EP-A-0335550 ist die Bildung eines biologisch aktiven lypolitischen Faktors, der mit der Tumorkachxie assoziiert ist, bekannt.

Aus EP-A-0311091 läßt sich die Kombination von Omega-3-Fettsäuren mit Triglyceriden entnehmen, die so einen erhöhten Schutz der Omega-3-Fettsäuren vor Oxidation und somit eine bessere Verfügbarkeit zur Bildung von ungesättigten Eicosanoiden gewährleistet.

Die tumorbedingte Auszehrung (= Kachexie) steht jedoch in keinerlei Zusammenhang mit chronisch-entzündlichen Prozessen oder etwa einem sog. "Postagressionsstoffwechsel" nach einem Trauma. Die zweite medizinische Anwendung von Omega-3-Fettsäuren in der Tumorkachexie ist für den Fachmann daher nicht offensichtlich und zeigt einen unerwartet positiven Effekt.

Eine Weiterbildung ist dadurch gekennzeichnet, daß 1,57 bis 50 g (Gramm), vorzugsweise 12,5 g, Omega-3-Fettsäuren auf ein oder mehrere für die Tagesdiät eines 175 cm (Zentimeter) großen Erwachsenen eingesetzt sind. Dabei werden vorzugsweise als Omega-3-Fettsäure Eicosapentaensäure ... (20:5 w-3), im folgenden kurz EPA genannt, und/oder Docosahexaensäure (22:6 w-3), im folgenden kurz DHA genannt, eingesetzt.

Diese Omega-3-Fettsäuren werden zur Kachexiebehandlung zweckmäßig in Form von Fischöl eingesetzt, das aus Fischfleisch, vorzugsweise von Kaltwasserfischen, insbesondere Lachs, Makrele, Dorsch, Salm (Atlantik), Sardine, Kabeljau und Hering, gewonnen ist.

Dieses Fischöl hat einen natürlichen hohen Gehalt an Omega-3-Fettsäuren und ist ein Nahrungsmittel, das ohne schädliche Nebenwirkungen in den hier in Frage stehenden Mengen aufgenommen werden kann.

Die besten antikachectischen Ergebnisse erzielt man, wenn die Omega-3-Fettsäure im Rahmen einer Diät eingesetzt wird, die extrem kohlenhydratarm ist und an verdaubarem Protein nur in ernährungsphysiologisch notwendiger Menge enthält. Eine dementsprechende Weiterbildung ist dadurch gekennzeichnet, daß der Einsatz im Rahmen einer oder mehrerer Nahrungspräparate erfolgt, die als Tagesdiät bestimmt sind zur ausschließlichen Ernährung einer 175 cm großen erwachsenen Person und die, abgesehen von Mineralstoffen, Vitaminen und dergleichen, gewichtsbezogene Gehälter wie folgt aufweisen: verdaubare Kohlehydraten minimal weniger als 30 g, verdaubares Protein in ernährungsphysiologisch ausreichender Menge von 50 bis 140 g, vorzugsweise 50 g, Fischöl mit einem Gesamtgehalt an Omega-3-Fettsäuren von 1,57 - 50 g, vorzugsweise 12,5 g, verdaubares anderes Fett, vorzugsweise in Form von mittelkettigen Fetten, in einer solchen Menge, daß zusammen mit dem eingesetzten Protein ein physiologisch ausreichender Gesamtverbrennungswert von 2000 bis 3000 Kcal (Kilokalorien), vorzugsweise 2500 Kcal, erzielt ist.

Mit einem solchen Präparat konnte eine signifikante Hemmung des Körpergewichtsverlustes bei Kachexie und in vielen Fällen eine Zunahme des gesamten Körperfettes und der trockenen Muskelmasse erzielt werden. Bemerkenswert ist, daß diese Ergebnisse erzielt werden mit einer für die normale, also nicht erhöhte, Kalorienzufuhr.

Die beobachtete Wirkung erwies sich auch spezifisch für die Omega-3-Fettsäure, denn vergleichbare Ergebnisse konnten mit einer kohlenhydratarmen Fettdiät, die keine Omega-3-Fettsäuren enthielt, nicht erzielt werden.

Kachexie tritt vor allem im Zusammenhang mit Colon-Adeno-Carcinom Typ (MAC 16) auf. Dieses Carcinom ist beschrieben in:
DOUBLE, J.A. and BIBBY, M.C.,
Characterisation and chemosensitivity of a well differentiated murine transplantable adenocarcinoma of the colon;
Br.J. Cancer 48, 739 - 742 (1983)
DOUBLE, J.A. and BELL, C.R.,
Chemotherapy of transplantable adenocarcinoma of the colon in mice;
Cancer Chemotherapy Rep. 59, 1083-1089 (1975)

Dieses Colon-Adeno-Carcinom Typ (MAC 16) ist resistent gegenüber den üblichen bekannten Chemotherapeutika.

Es hat sich gezeigt, daß der Einsatz der Omega-3-Fettsäuren zur Bekämpfung der vom Colon-Adeno-Carcinom Typ (MAC 16) hervorgerufenen Kachexie neben der Hemmung des kachexiebedingten Körpergewichtsverlustes auch zu einer signifikanten Hemmung der Tumorwachstumsrate und sogar zu einer Tumorgewichtsreduzierung führen kann.

Das ist ein quasi synergistischer Effekt, weil die Hemmung des Gewichtsverlustes größer war als die erzielte Tumorgewichtsreduzierung.

Die antikachektische Wirkung der Omegasäuren ist weiterhin von Bedeutung dadurch, daß durch Erhaltung des Ernährungs-und Kräftezustandes des Patienten andere chemotherapeutische oder physikalische Maßnahmen zur Tumortherapie begleitend durchgeführt werden können bei verbesserter Verträglichkeit.

Für die Kachexiebekämpfung wird vorzugsweise das Antikachectikum in Form eines diätischen Nahrungsmittels bereitgestellt und es ist Aufgabe einer Weiterbildung der Erfindung, ein solches Nahrungsmittel so auszugestalten, daß es möglichst angenehm per os zu applizieren ist.

Ein solches Nahrungsmittel ist vorzugsweise dadurch gekennzeichnet, daß die Inhaltsstoffe vermischt und zu einem in Wasser aufschlämmbaren Pulver versprüht sind.

Es hat sich gezeigt, daß in so pulverisierter Form das Diätetikum einen tragbaren Geschmack hat, der im übrigen durch Zugabe von Obstsäften oder Obstbrei noch maskiert werden kann.

Ein diätetisches Nahrungsmittel kann auch so gestaltet werden, daß die Tagesdosis Fischöl getrennt von der Einnahme der versprühten und dann aufgeschlämmten Form der Eiweiß-Fett-Komponenten löffelweise oder in Form von verdaubaren Kapseln appliziert wird.

Es empfiehlt sich, das diätetische Nahrungsmittel in Form von Tagesportionen bereitzustellen, wobei eine jede Tagesportion als ausschließliches Nahrungsmittel vorgesehen ist, abgesehen von nötigen Zusätzen an Vitaminen, Mineralstoffen, Spurenelementen und eventuellen Geschmacksstoffen. Entsprechende Ausgestaltungen sind Gegenstand der weiteren Unteransprüche.

Es werden nun einige Ausführungsbeispiele der Erfindung beschrieben.

BEISPIEL 1

Aus frisch gefangenen B = Heringen werden sämtliche Eingeweide einschließlich der Leber entnommen. Die so ausgenommenen Fischkörper werden zerkleinert, dann verbreit und in ein Sieb gepreßt. Die durch das Sieb tretende Flüssigkeit wird anschließend zentrifugiert und das darin enthaltene Fischöl wird abgeschichtet und dekantiert.

B1 = 41,76 g dieses so gewonnenen Fischöls enthalten B2 = 7,5 g EPA und B3 = 5 g DHA und haben einen ernährungsphysiologischen Brennwert von B4 = 375 Kcal.

Diese 41,76 g Fischöl werden mit C = 201,93 g Speisefett, D = 32,97 g Säurecasein, E = 7,69 g Sojaeiweiß, F = 42,86 g Magermilchpulver und G = 0,16 g Tocopherolacetat als Stabilisator sowie P = 480 ccm (Kubikzentimeter) Wasser vermischt. Die gesamte Masse wird dann homogenisiert, so daß man eine Emulsion erhält.

Das für die Emulsion eingesetzte Speisefett ist C1 = Myritol® 318 mit einem Kalorienwert von C2 = 1817,28 Kcal für die eingesetzte Menge.

Das eingesetzte Säurecasein hat einen Proteingehalt von 91%, so daß der Proteingehalt der eingesetzten Menge D1 = 30 g beträgt. Der ernährungsphysiologische Brennwert der eingesetzten Menge Säurecasein beträgt D2 = 124,64 Kcal.

Das eingesetzte Sojaeiweiß hat einen Proteingehalt von 65 %, das entspricht für die eingesetzte Menge einem Proteingehalt von E1 = 5,0 g, der einen ernährungsphysiologischen Brennwert von E2 = 25,57 Kcal hat.

Das eingesetzte Magermilchpulver hat einen Proteingehalt von 35 %, so daß der Proteingehalt der eingesetzten Menge F1 = 15 g und der ernährungsphysiologische Brennwert F2 = 157,72 Kcal beträgt.

Die Emulsion wird auf eine um eine vertikale Achse rotierende Sprühscheibe gegossen, die am oberen Ende eines Sprühturms angeordnet ist. Die Scheibe rotiert mit etwa 10.000 Umdrehungen pro Minute. In den Sprühturm wird von unten heiße Luft eingeströmt mit einer Temperator von I3 = 200°C. Die Luft verläßt den Sprühturm oben mit einer Temperatur von I4 = 105°C. Die versprühte Emulsion bildet ein sprühgetrocknetes Pulver, das nach unten abrieselt. Das Schüttgewicht des fertigen Pulvers beträgt S = 500 g pro Liter.

30 g des gewonnenen Pulvers werden in 100 ccm Wasser, Milch oder Fruchtsaft emulgiert und können dann getrunken werden. Der Geschmack dieses Getränks ist nicht unangenehm.

Das Pulver wird verabfolgt in einer Tagesdosis von H4 = 327,11 g für eine 175 cm große Person. Diese Tagesdosis enthält H1 = 244 g Fett, H2 = 50 g Protein und H3 = 24 g Kohlenhydrate. Der ernährungsphysiologische Brennwert der Tagesdosis beträgt H5 = 2500 Kcal.

Die Tagesdosis von H4 Gramm, die auf eine 175 cm große Person bemessen ist, wird im proportionalen Verhältnis zur Körperlänge modifiziert verabfolgt. Die Verabfolgung erfolgt über einen Zeitraum von L = 30 Tagen.

Nach der Behandlungsdauer von L = 30 Tagen ist eine signifikante Körpergewichtszunahme und eine signifikante Reduktion des Tumorgewichtes der behandelten Person aufgrund der Ergebnisse von Tierversuchen zu erwarten.

Die nachfolgenden Beispiele 2 bis 7 unterscheiden sich vom Beispiel 1 nur durch die aus der nachfolgenden TABELLE 1 ersichtliche Tatsachen.

TABELLE 1

| Beispiel | 1 | 2 | 3 | 4 |
|---|---|---|---|---|
| B | Hering | Makrele | Hering | Sardine |
| B1 | 41,67 | 69,44 | 97,22 | 69,44 |
| B2 | 7,5 | 12,4 | 17,5 | 12,5 |
| B3 | 5,0 | 8,33 | 11,67 | 8,33 |
| B4 | 375,0 | 625,0 | 875,0 | 625,0 |
| C | 201,93 | 174,15 | 146,37 | 174,15 |
| C1 | Myritol 318 | Myritol 318 | Myritol 318 | Myritol 318 |
| C2 | 1817,28 | 1567,35 | 1317,33 | 1567,35 |
| D | 32,97 | 32,97 | 32,97 | 32,97 |
| D1 | 30,0 | 30,0 | 30,0 | 30,0 |
| D2 | 124,46 | 124,46 | 124,46 | 124,46 |
| E | 7,69 | 7,69 | 7,69 | 7,69 |
| E1 | 5,0 | 5,0 | 5,0 | 5,0 |
| E2 | 25,57 | 25,57 | 25,57 | 25,57 |

TABELLE 1 - Fortsetzung

| Beispiel | 1 | 2 | 3 | 4 |
|---|---|---|---|---|
| F | 42,86 | 42,86 | 42,86 | 42,86 |
| F1 | 15,0 | 15,0 | 15,0 | 15,0 |
| F2 | 157,72 | 157,72 | 157,72 | 157,72 |
| G | 0,16 | 0,16 | 0,16 | 0,16 |
| H1 | 244 | 244 | 244 | 244 |
| H2 | 50 | 50 | 50 | 50 |
| H3 | 24 | 24 | 24 | 24 |
| H4 | 327,11 | 332,31 | 332,31 | 331,31 |
| H5 | 2500 | 2500 | 2500 | 2500 |
| I3 | 210 | 210 | 210 | 210 |
| I4 | 104 | 104 | 104 | 104 |
| L | 30 | 30 | 30 | 30 |
| P | 480 | 480 | 480 | 480 |
| S | 500 | 500 | 500 | 500 |

TABELLE 1 - Fortsetzung

| Beispiel | 5 | 6 | 7 |
|---|---|---|---|
| B | Sardine | Sardine | Makrele |
| B1 | 35,71 | 52,82 | 11,43 |
| B2 | 10,0 | 15 | 3,2 |
| B3 | 2,04 | 3,06 | 0,7 |
| B4 | 321,39 | 475,38 | 102,87 |
| C | 208,29 | 191,2 | 232,57 |
| C1 | Myritol®<br>318 | Myritol®<br>318 | Myritol®<br>318 |
| C2 | 1874,61 | 1720,8 | 2093,13 |
| D | 32,97 | 32,97 | 32,97 |
| D1 | 30,0 | 30,0 | 30,0 |
| D2 | 124,46 | 124,46 | 124,46 |
| E | 7,69 | 7,69 | 7,69 |
| E1 | 5,0 | 5,0 | 5,0 |
| E2 | 25,57 | 25,57 | 25,57 |

TABELLE 1 - Fortsetzung

| Beispiel | 5 | 6 | 7 |
|----------|--------|--------|--------|
| F | 42,86 | 42,86 | 42,86 |
| F1 | 15,0 | 15,0 | 15,0 |
| F2 | 157,72 | 157,72 | 157,72 |
| G | 0,16 | 0,16 | 0,03 |
| H1 | 244 | 244 | 244 |
| H2 | 50 | 50 | 50 |
| H3 | 24 | 24 | 24 |
| H4 | 332,00 | 332,00 | 332,00 |
| H5 | 2500 | 2500 | 2500 |
| I3 | 210 | 210 | 210 |
| I4 | 104 | 104 | 104 |
| L | 60 | 30 | 90 |
| P | 480 | 480 | 480 |
| S | 500 | 500 | 500 |

Myritol® 318, wie unter C1 angegeben ist ein eingetragenes Warenzeichen, unter dem ein Triglycerid bekannt ist, das wie folgt charakterisiert ist.

Myritol® 318 ist zusammengesetz aus: Capryl-/Caprinsäure, Triglycerid.

Beschaffenheit von Myritol® 318: Es ist ein neutrales, fast farbloses, geruchsfreies, klares Öl mit niedriger Viskosität.

Die Kenndaten von Myritol® 318 sind in der nachfolgenden TABELLE 2 angegeben:

8

TABELLE 2

| Kenndaten von Myritol 318 | |
|---|---|
| Säurezahl | unter 0,1 |
| Verseifungszahl | 340 - 350 |
| Jodzahl | ca. 0,5 |
| Hydroxylzahl | unter 5 |
| Brechungsindex (20°C) | 1,448 - 1,450 |
| Trübungspunkt | unter -5°C |
| Stockpunkt | unter -10°C |
| Dichte (20°C) | 0,945 - 0,947 g/cm$^3$ |
| Viskosität (20°C) | 25 - 31 mPa.s |

Die Beispiele 1 bis 7 sind abänderbar, indem die eingesetzten B = Gramm Fischöl nicht mit in die Emulsion eingemischt werden. Sie sind demzufolge auch nicht in dem aus der Emulsion gewonnenen Pulver enthalten. Die B = Gramm Fischöl werden zusätzlich zu dem gewonnenen fischölfreien Pulver oral appliziert, und zwar unter Beibehalt der Einsatzmengen aus TABELLE 1.

Bei Verabfolgung der nach der Körpergröße modifizierten Tagesdosis aus den Beispielen an Personen, die vom Colon-Adeno-Carcinom Typ (MAC 16) befallen sind und infolge dessen unter Kachexie leiden, ist nach der unter L angegebenen Behandlungsdauer eine Zunahme des Körpergewichtes und eine Abnahme des Tumors zu erwarten. Diese Annahme gründet sich ebenfalls auf Ergebnisse von durchgeführten Tierversuchen.

Bei Verabfolgung der nach der Körpergröße modifizierten Tagesdosis aus den Beispielen an Personen, die unter Kachexie leiden, die jedoch durch einen anderen Tumor als den Colon-Adeno-Carcinom Typ (MAC 16) hervorgerufen ist, ist nach der unter L angegebenen Behandlungsdauer eine Zunahme des Körpergewichtes und eine Abnahme des Tumorgewichtes zu erwarten. Diese Annahme gründet sich ebenfalls auf Ergebnisse von durchgeführten Tierversuchen und ist in Verbindung mit dem Colon-Adeno-Carcinom Typ (MAC 13) bereits nachgewiesen.

Die Verabfolung der nach der Körpergröße modifizierten Tagesdosis aus den Beispielen an Personen, die unter Kachexie leiden, die nicht durch einen Tumor bedingt ist, ist nach der unter L angegebenen Behandlungsdauer eine Zunahme des Körpergewichtes zu erwarten. Diese Annahme gründet sich ebenfalls auf die Ergebnisse von durchgeführten Tierversuchen.

**Patentansprüche**

1.  Verwendung von Omega-3-Fettsäuren enthaltendem Fischöl zur Herstellung eines Antikachektikums zur Behandlung von Tumorkachexie (Kachexial cancerativa).

2.  Verwendung nach Anspruch 1, gekennzeichnet durch den Einsatz von Fischöl, das aus dem Fischfleisch von Kaltwasserfischen, insbesondere Lachs, Makrele, Dorsch, Salm (Atlantik), Sardine, Kabeljau und Hering, gewonnen ist.

3.  Verwendung nach Anspruch 1 oder 2, dadurch gekennzeichnet,
    daß 1,57 bis 50 g (Gramm), vorzugsweise 12,5 g, Omega-3-Fettsäure für die Tagesdiät eines 175 cm (Zentimeter) großen Erwachsenen eingesetzt wsind.

4.  Verwendung nach einem der vorhergehenden Ansprüche, dadurch gekennzeichnet,
    daß als Omega-3-Fettsäure Eicosapentaensäure (20:5 w-3), im folgenden kurz EPA genannt, und/oder Docosahexaensäure (22:6 w-3), im folgenden kurz DHA genannt, eingesetzt wird.

5.  Verwendung nach einem der vorhergehenden Ansprüche, gekennzeichnet durch
    den Einsatz im Rahmen einer oder mehrerer Nahrungspräparate, die als Tagesdiät bestimmt sind zur ausschließlichen Ernährung einer 175 cm großen erwachsenen Person und die, abgesehen von Mineralstoffen, Vitaminen und dergleichen, gewichtsbezogene Gehälter wie folgt aufweisen:
    verdaubare Kohlehydrate minimal weniger als 30 g,
    verdaubares Protein in ernährungsphysiologisch ausreichender Menge von 50 bis 140 g, vorzugsweise 50 g,

Fischöl mit einem Gesamtgehalt an Omega-3-Fettsäuren von 1,57 - 50 g, vorzugsweise 12,5 g,

verdaubares anderes Fett, vorzugsweise in Form von mittelkettigen Fetten, in einer solchen Menge, daß zusammen mit dem eingesetzten Protein ein physiologisch ausreichender Gesamtverbrennungswert von 2000 bis 3000 Kcal (Kilokalorien), vorzugsweise 2500 Kcal, erzielt ist.

6. Verwendung nach einem der vorhergehenden Ansprüche in Form eines diätetischen Nahrungsmittels, dadurch gekennzeichnet,

daß die Inhaltsstoffe vermischt und zu einem in Wasser aufschlämmbaren Pulver versprüht sind.

7. Verwendung nach einem der vorhergehenden Ansprüche in Form eines diätetischen Nahrungsmittels, dadurch gekennzeichnet,

daß auf 100 Teile Protein 480 bis 500 Teile, vorzugsweise 490 Teile, Fett einschließlich Fischöl und 40 bis 60 Teile, vorzugsweise 48 Teile, Kohlehydrate eingesetzt sind.

8. Verwendung nach einem der vorhergehenden Ansprüche in Form eines diätetischen Nahrungsmittels, gekennzeichnet durch

13 - 30 % Fischöl, mittelkettige Fette bis auf einen Gesamtfettgehalt von 74 %,, 8 bis 20 %, vorzugsweise 15 %, Protein und 7 bis 10 %, vorzugsweise 7 %, Kohlehydrate und dadurch,

daß ein Fischöl eingesetzt ist, das 50 bis 12 %, vorzugsweise 18 %, EPA und 5 bis 20 %, vorzugsweise 12 %, DHA enthält.

9. Verwendung nach einem der Ansprüche 6 bis 8 in Form eines diätetischen Nahrungsmittels, dadurch gekennzeichnet,

daß das Fischöl gewonnen ist aus dem Fleischvon Kaltwasserfischen, vorzugsweise Lachs, Makrele, Dorsch, Salm (Atlantik), Sardine, Kabeljau und Hering.

10. Verwendung nach einem der Ansprüche 6 bis 9 in Form eines diätetischen Nahrungsmittels, dadurch gekennzeichnet,

daß 9 - 11 %, vorzugsweise 10 %, des Proteins in Form von Sojaeiweiß,

daß 49 - 55 %, vorzugsweise 50 %, des Proteins in Form von Magermilchpulver und

daß der Rest auf 100 % Protein in Form von Säurecasein eingesetzt ist.

**Claims**

1. Use of fish oil containing omega-3-fatty acids for producing an anti-cachechtic agent for the treatment of cancerous cachexia

2. Use according to claim 1, characterized by the use of fish oil obtained from the meat of cold water fish, particularly salmon, mackerel, codling, Atlantic salmon, sardines, cod and herring.

3. Use according to claims 1 or 2, characterized in that 1.57 to 50 g (gram), preferably 12.5 g of omega-3-fatty acid are used for the daily diet of a 175 cm (centimetre) adult.

4. Use according to one of the preceding claims, characterized in that as omega-3-fatty acid use is made of eicosapentaenic acid (20:5 w-3), hereinafter called EPA for short, and/or docosahexaenic acid (22:6 w-3), hereinafter called DHA for short.

5. Use according to one of the preceding claims, characterized by use within the scope of one or more food preparations which, as the daily diet, are intended for the exclusive feeding of a 175 cm adult and which, apart from mineral substances, vitamins and the like, have the following weight-related contents: digestible carbohydrates min. less than 30 g,
digestible protein in nutrition physiologically adequate quantities of 50 to 140 g, preferably 50 g,
fish oil with a total omega-3-fatty acid content of 1.57 to 50 g, preferably 12.5 g,
digestible other fat, preferably in the form of medium-chain fats, in a quantity such that together with the protein used a physiologically adequate total metabolization value of 2000 to 3000 Kcal (kilocalories), preferably 2500 Kcal is obtained.

6. Use according to one of the preceding claims in the form of a dietetic food, characterized in that the constituents are mixed and sprayed to a powder suspendable in water.

7. Use according to one of the preceding claims in the form of a dietetic food, characterized in that 480 to 500 parts and preferably 490 parts of fat, including fish oil, and 40 to 60 parts, preferably 48 parts of carbohydrates are used per 100 parts of protein.

8. Use according to one of the preceding claims in the form of a dietetic food, characterized by 13 to 30% fish oil, medium-chain fats up to a total fat content of 74%, 8 to 20% and preferably 15% protein and 7 to 10% and preferably 7% carbohydrates and by the use of a fish oil containing 50 to 12% and preferably 18% EPA and 5 to 20%, preferably 12% DHA.

9. Use according to one of the claims 6 to 8 in the form of a dietetic food, characterized in that fish oil is obtained from the meat of cold water fish, preferably salmon, mackerel, codling, Atlantic salmon, sardines, cod and herring.

10. Use according to one of the claims 6 to 9 in the form of a dietetic food, characterized in that 9 to 11% and preferably 10% of the protein is in the form of soybean protein, 49 to 55% and preferably 50% of the protein is in the form of skim milk powder and the remainder up to 100% protein is in the form of acid casein.

**Revendications**

1. Utilisation d'une huile de poisson contenant des oméga-3-acides gras pour fabriquer un produit anticachexie pour traiter de la cachexie à tumeur ("Kachexial cancerativa").

2. Utilisation selon la revendication 1, caractérisée par l'utilisation d'une huile de poisson obtenue à partir de la chair de poissons d'eau froide, notamment du saumon, de maquereaux, de petite morue, de saumon (de l'Atlantique), de sardines, de cabillaud et de hareng.

3. Utilisation selon la revendication 1 ou 2, caractérisée en ce que l'on utilise 1,57 à 50 g (grammes), avantageusement 12,5 g d'oméga-3-acide gras pour la ration quotidienne d'un adulte de 175 cm (centimètres) de taille.

4. Utilisation selon l'une des revendications précédentes, caractérisée en ce que l'on utilise comme oméga-3-acide gras l'acide eicosapentaénoïque (20;5 w-3), désigné ci-après- par l'abréviation EPA, et/ou l'acide docosahexaénoique (22:6 w-3), désigné ci-après par l'abréviation DHA.

5. Utilisation selon l'une des revendications précédentes, caractérisée par l'utilisation, dans le cadre d'une ou plusieurs préparations nutritives, qui sont déterminées comme rations quotidiennes pour l'alimentation exclusive d'une personne adulte de 175 cm de taille et qui, à part les substances minérales, les vitamines et substances analogues, présentent les teneurs pondérales suivantes:
   - hydrates de carbone digestibles, au minimum moins de 30 g,
   - protéines digestibles, en une quantité suffisantes pour être physiologiquement nutritives, de 50 à 140 g, avantageusement de 50 g,
   - huile de poisson en une quantité totale d'oméga-3 acides gras de 1,57 à 50 g, avantageusement de 12,5g,
   - autre matière grasse digestible, avantageusement sous forme de matière grasse à chaîne de longueur moyenne, en une quantité telle qu'avec la protéine utilisée, on atteigne une valeur calorique totale suffisante physiologiquement de 2000 à 3000 Kcal (kilocalories), avantageusement de 2 500 Kcal.

6. Utilisation selon l'une des revendications précédentes, sous forme d'un aliment diététique, caractérisée en ce que les constituants sont mélangés et atomisés en une poudre délayable dans de l'eau.

7. Utilisation selon l'une des revendications précédentes sous forme d'un aliment diététique, caractérisée en ce que, pour 100 parties de protéines, on utilise 480 à 500 parties avantageusement 490 parties de matière grasse y compris l'huile de poisson, et 40 à 60 parties, avantageusement 48 parties, d'hydrates de carbone.

8. Utilisation selon l'une des revendications précédentes sous forme d'un aliment diététique, caractérisée en ce que l'aliment contient 13 à 30 % d'huile de poisson, des matières grasses à longueur moyenne de chaîne jusqu'à une teneur totale de 74 %, 8 à 20 %, avantageusement 15 %, de protéines et 7 à 10 %, avantageusement 7 % d'hydrates de carbone et en ce que l'on utilise une huile de poisson qui contient 50 à 12 %, avantageusement 18 %, de EPA et 5 à 20 %, avantageusement 12 %, de DHA.

9. Utilisation selon l'une des revendications 6 à 8 sous forme d'un aliment diététique, caractérisée en ce que l'huile de poisson est obtenue à partir de la chair de poissons d'eau froide, avantageusement du saumon, des maquereaux, de la petite morue, du saumon (de l'Atlantique), des sardines, du cabillaud et du hareng.

10. Utilisation selon l'une des revendications 6 à 9, sous forme d'un aliment diététique, caractérisée en ce qu'on utilise 9 à 11 %, avantageusement 10 %, de la protéine sous forme d'albumine de soja, 49 à 55 %, avantageusement 50 % de la protéine sous forme de poudre de lait écrémé, et
en ce que le reste, pour compléter à 100 % de protéine, est sous forme de caséine acide.